# EUROPEAN PATENT APPLICATION

(11) **EP 4 596 682 A1**
(43) Date of publication of application: **06.08.2025**
(21) Application number: 23871623.7
(22) Date of filing: 27.08.2023
(51) Int. Cl.: C12N 5/0797, A61K 35/28, A61K 35/30, A61P 37/04, A61P 43/00, C12N 5/0775, C12N 5/0783

(54) **FINE PARTICLES, NK CELL ACTIVATOR, CULTURE METHOD FOR NK CELLS, PRODUCTION METHOD FOR ACTIVATED NK CELLS, AND ACTIVATION METHOD FOR NK CELLS**

(30) Priority: 28.09.2022 JP 2022155607
(71) Applicant: Dexon Pharmaceuticals Inc., Tokyo, 103-0027 (JP)
(72) Inventor: KOGA Shoji, Tokyo 102-0082 (JP)
(74) Representative: Godemeyer Blum Lenze Patentanwälte Partnerschaft mbB - werkpatent
(86) International application number: PCT/JP2023/030834
(87) International publication number: WO 2024/070382

(57) **Abstract**

Microparticles comprising miRNA with respect to the activation of NK cells and derived from culture supernatant of dental pulp-derived stem cells or culture supernatant of adipose-derived stem cells are new microparticles that enables controlling the expression of proteins and/or genes related to the activation of NK cells; an agent for activating NK cells; a method for culturing NK cells; a method for producing activated NK cells; NK cells; a method for activating NK cells NK cells.

## Description

### Technical Field

The present invention relates to microparticles, an agent for activating NK cells, a method for culturing NK cells, a method for producing activated NK cells, and a method for activating NK cells.

### Background Art

NK (natural killer) cells, which have the function of finding and destroying virus-infected cells or cancer cells, are attracting attention as a decisive method for preventing infectious diseases such as influenza or preventing cancer.

The activity (destruction ability) of NK cells deteriorates with age, and the incidence of cancer increases with age, which is supposed to be related to the decline in activation of NK cells. Since NK cells are affected by the autonomic nerves, stress leads to sympathetic dominance to deteriorate the activity (destruction ability) of NK cells sharply. Thus, the activation of NK cells is essential for health maintenance.

The exploration of substances that promote the activation of NK cells has recently made progress.

For example, Patent Literature 1 describes a method for increasing the number of NK cells, comprising contacting at least one NK cell with at least one NK-stimulating exosome comprising one or more stimulatory peptides present on the exosome membrane, wherein the exosome is an extracellular product from exosome-secreting cells.

Meanwhile, the investigation of microRNAs (miRNAs) has progressed to clarify the functions of miRNAs related to NK cells and objective genes thereof (see Non Patent Literatures 1 and 2).

Non Patent Literature 1 discloses that the expression of miR-155 is regulated by stimulation (activation of IL-12, IL18, CD16, or others) that strongly induces IFN-y in NK cells. It is also disclosed that miR-155 functions as a positive regulatory factor of production of IFN-y in human NK cells at least partially through downregulation of 5'-inositol phosphatase (SHIP1).

Non Patent Literature 2 discloses that the survival, the expansion, and the activation of NK cells and the enhancement of tumor control of NK cells due to overexpression of miR-155 in the NK cells can be partially explained by a decrease in expression of the inositol phosphatase SHIP1 and increase in activation of ERK and AKT kinase. It is also disclosed that the regulation of miR-155 is important for the activation of NK cells.

### Document List

### Patent Literature

Patent Literature 1: Japanese Patent Application Laid-Open No. 2022-043068 Non Patent Literatures

Non Patent Literature 1: Blood (2012) 119 (15): 3478-3485
Non Patent Literature 2: Blood (2013) 121 (16): 3126-3134

### Summary of Invention

### Technical Problem

Patent Literature 1 has not described miRNA.

Although Non Patent Literatures 1 and 2 have described the function and the objective gene of a specific miRNA, namely miR-155, in NK cells, Non Patent Literatures 1 and 2 have not clearly described exosomes derived from mesenchymal stem cells as means for administering this miRNA.

The present invention is directed to providing new microparticles that enables controlling the expression of a protein and/or a gene related to the activation of NK cells.

### Solution to Problem

The present inventors have found that microparticles containing a specific microRNA enable controlling the expression of a protein and/or a gene related to the activation of NK cells.

The present invention and preferable configurations of the present invention are specifically as follows.

[1] Microparticles, comprising miRNA related to activation of NK cells,
   wherein the microparticles are derived from culture supernatant of dental pulp-derived stem cells or culture supernatant of adipose-derived stem cells.
[2] The microparticles according to [1], wherein the microparticles are exosomes.
[3] The microparticles according to [1] or [2], wherein the microparticles are an agent for promoting expression of granzyme B.
[4] The microparticles according to any one of [1] to [3], wherein the microparticles are an agent for controlling expression of SHIP1, and comprise hsa-miR-155-5p as miRNA targeting a gene concerning expression of SHIP1.
[5] The microparticles according to any one of [1] to [4], wherein the microparticles are isolated by purification from the culture supernatant of the dental pulp-derived stem cells or the culture supernatant of the adipose-derived stem cells.
[6] The microparticles according to [5], wherein the microparticles do not comprise components of the culture supernatant of the dental pulp-derived stem cells or the culture supernatant of the adipose-derived stem cells other than the microparticles.
[7] The microparticles according to any one of [1] to [6], comprising hsa-miR-155-5p at higher concentration than the culture supernatant of the dental pulp-derived stem cells or the culture supernatant of the adipose-derived stem cells.
[8] An agent for activating NK cells, comprising the microparticles according to any one of [1] to [7].
[9] A method for culturing NK cells, wherein NK cells are cultured in medium for activating NK cells comprising an effective amount of the microparticles according to any one of [1] to [7] or an effective amount of the agent for activating NK cells according to [8].
[10] A method for producing activated NK cells, comprising administering an effective amount of the microparticles according to any one of [1] to [7] or an effective amount of the agent for activating NK cells according to [8] to a composition comprising NK cells.
[11] NK cells
   cultured in medium for activating NK cells comprising an effective amount of the microparticles according to [1] or an effective amount of the agent for activating NK cells according to [8]; or
   produced by administering an effective amount of the microparticles according to any one of [1] or an effective amount of the agent for activating NK cells according to [8] to a composition comprising the NK cells.
[12] A method for activating NK cells, comprising administering an effective amount of the microparticles according to any one of [1] to [7] or an effective amount of the agent for activating NK cells according to [8] to a subject.
[13] The method for activating NK cells according to [12], wherein the subject is a non-human animal.
[14] The method for activating NK cells according to [12] or [13], wherein the number of the microparticles per one NK cell is 50 or more.

### Effects of Invention

According to the present invention, new microparticles can be provided that enables controlling the expression of a protein and/or a gene.

### Brief Description of Drawings

[FIG. 1] A schematic diagram showing modeled function of NK cells in the induction of miR-155 and the regulation of production of IFN-y after the activation of the NK cells.
[FIG. 2] A graph showing the expression levels of granzyme B when the number of exosomes for treatment derived from various mesenchymal stem cells per NK cell was changed.

### Description of Embodiments

Hereinafter, the present invention will be described in detail.

Although components described below may be explained based on representative embodiments and specific examples, the present invention is not limited by such embodiments. A numerical range indicated with the term "to" herein means a range including numerical values described before and after the term "to" as the lower limit and the upper limit.

### [Microparticles]

Microparticles of the present invention contain miRNA related to the activation of NK cells, and are derived from supernatant of dental pulp-derived stem cells or supernatant of adipose-derived stem cells.

The microparticles of the present invention enables controlling the expression of a protein and/or a gene related to the activation of NK cells. Consequently, the microparticles of the present invention enables activating NK cells.

Hereinafter, preferable aspects of the microparticles of the present invention will be described.

### <Activation of NK cells>

NK cells, which are one type of lymphocytes, account for 10 to 30% of lymphocytes in blood of humans. NK cells, which are immunocytes naturally inherent in animals such as humans, contribute to natural immunity.

NK cells usually have the function of finding and destroying virus-infected cells and cancer cells. NK cells have activating receptors and inhibiting receptors. NK cells encounter normal cells, resulting in the dominance of signals from inhibiting receptors, so that the NK cells do not attack the normal cells. Since NK cells have various receptors that can capture abnormal cells, NK cells can capture abnormal cells even without a specific antigen to immediately remove the abnormal cells.

NK cells are activated in case of emergency to induce the activity of acquired immunocytes (dendritic cells, T cells, and B cells), resulting in attacking virus-infected cells or others, so that the immune reaction and the inflammatory reaction can be regulated. Specifically, NK cells find virus-infected cells or cancer cells, so that signals from activating receptors enter to activate the NK cells, which attack virus-infected cells or cancer cells. The activated NK cells synthesize cytotoxic factors such as perforin (protein that makes pores in cell membranes of target cells) and granzymes (a group of serine proteases that induces cell death in target cells) to extracellularly secrete the cytotoxic factors and destroy abnormal cells. The NK cells secrete various cytokines and chemokines such as IFN-y and TNF-alpha to directly act on dendritic cells, resulting in regulating and activating acquired immunocytes.

Five granzymes are present in humans. Ten granzymes are present in mice. Among granzymes, granzyme B is the most abundantly present in humans. That is, granzyme B is a serine protease that is the most commonly present in granules of NK cells and cytotoxic T cells. Granzyme B is secreted with perforin from these cells to mediate apoptosis of target cells. Granzyme B has various subsidiary functions. Granzyme B stimulates the release of cytokines to partake in induction of inflammation, and also partakes in the remodeling of the extracellular matrix. It has been suggested that the level of granzyme B partakes in many autoimmune diseases, several skin diseases, and type I diabetes.

The activity of NK cells varies depending on the lifestyle. For example, the activity of NK cells can be enhanced depending on meals or manners of living. Meanwhile, factors such as fatigue or stress, irregular hours, and inadequate exercise deteriorate the activity of NK cells.

The activity of NK cells herein is quantitatively determined based on the expression level of granzyme B. The expression level of granzyme B can be measured by a known method, and may be measured with a commercially available kit. For example, SensoLyte 520 Granzyme B Activity Assay Kit, Fluorimetric (available from AnaSpec) (SensoLyte is a registered trademark) can be used.

A common concentration of granzyme B (in blood plasma) is 20 to 40 pg/ml. The microparticles of the present invention enables activating NK cells to increase the expression level of granzyme B to 41 pg/ml or more. It is preferable that the expression level can be increased to 50 pg/ml or more, it is more preferable that the expression level can be increased to 60 pg/ml or more, and it is particularly preferable that the expression level can be increased to 70 pg/ml or more.

It is thus preferable that the microparticles of the present invention be an agent for promoting the expression of granzyme B.

### <Details of microparticles>

The microparticles of the present invention contain miRNA related to the activation of NK cells, and are derived from culture supernatant of dental pulp-derived stem cells or culture supernatant of adipose-derived stem cells. Hereinafter, culture supernatant of dental pulp-derived stem cells and culture supernatant of adipose-derived stem cells are also referred to as "dental pulp-derived stem cells or other cells".

The miRNA is contained in the microparticles of the present invention as the active ingredient.

The microparticles of the present invention are derived from the dental pulp-derived stem cells or other cells by secretion, budding, or dispersion from mesenchymal stem cells such as dental pulp-derived stem cells to be exuded, released, or shed into cell culture medium. The microparticles are contained in (derived from) culture supernatant of dental pulp-derived stem cells or culture supernatant of adipose-derived stem cells. It is preferable that the microparticles be derived from culture supernatant of dental pulp-derived stem cells. The microparticles derived from culture supernatant of dental pulp-derived stem cells or other cells does not need to be necessarily acquired from the culture supernatant of the dental pulp-derived stem cells or other cells. For example, if microparticles isolated from inside dental pulp-derived stem cells by any method are the same as the microparticles that can be isolated from culture supernatant of dental pulp-derived stem cells, the microparticles isolated from inside dental the pulp-derived stem cells are also the microparticles derived from the culture supernatant of the dental pulp-derived stem cells.

The microparticles derived from the culture supernatant of the dental pulp-derived stem cells or other cells may be used as the culture supernatant containing the microparticles, or the microparticles derived from culture supernatant of dental pulp-derived stem cells may be purified from the culture supernatant for use. It is preferable that the microparticles be purified from the culture supernatant.

In the present invention, it is more preferable that the microparticles be isolated by purification from the culture supernatant of the dental pulp-derived stem cells or the culture supernatant of the adipose-derived stem cells. It is particularly preferable that the microparticles or the microparticle composition do not contain components of the culture supernatant of dental the pulp-derived stem cells or the culture supernatant of the adipose-derived stem cells other than the microparticles.

The origin of the microparticles can be discriminated by a known method. For example, it can be determined which stem cells among dental pulp-derived stem cells, adipose-derived stem cells, bone marrow-derived stem cells, umbilical cord-derived stem cells, and other stem cells are the origin of the microparticles by the method described in J Stem Cell Res Ther (2018) 8:2. Specifically, the origin of the microparticles can be determined based on the miRNA pattern of the microparticles.

### (miRNA)

In the present invention, the microparticles contain miRNA related to the activation of NK cells.

In the present invention, the miRNA (microRNA) is an RNA molecule having 21 to 25 bases (nucleotides). The microRNA enables decomposing target gene mRNA or suppressing mRNA in the decoding or transcription step to regulate gene expression.

In the present invention, miRNA may be a single strand (monomer) or a double strand (dimer). In the present invention, it is preferable that miRNA be mature miRNA obtained by cleaving miRNA with ribonuclease such as Dicer.

The sequences of the miRNAs such as hsa-miR-155-5p described herein are registered in association with the accession numbers thereof in known databases (for example, miRBase database). Those skilled in the art can determine the sequences thereof unambiguously. For example, the accession number of hsa-miR-155-5p is MIMAT0000646, and the sequence thereof is registered in the miRBase database. Hereinafter, the accession numbers of the miRNAs are omitted.

The miRNA in the present invention however includes variants in which around one to five bases are different from that or those of mature miRNA such as hsa-miR-155-5p. The miRNAs in the present invention include polynucleotides consisting of nucleotide sequences having identity to the nucleotide sequences of the miRNAs (for example, hsa-miR-155-5p) or the nucleotide sequences complementary thereto and having the function of the miRNAs in the present invention. The "identity" is the degree of identity between sequences to be compared that are appropriately aligned, and means the incidence (%) of amino acids or nucleic acids of the one sequence that are exactly identical to those of the other sequence. Sequences can be aligned, for example, by any algorithm such as BLAST. For example, the identity is 80%, 85%, 90%, 91%, 92%, 93%, 94%, 95%, 96%, 97%, 98%, or around 99%. For example, the polynucleotides consisting of nucleotide sequences having identity may have point mutations, deletions, and/or additions in the nucleotide sequences of the miRNAs. The number of bases of point mutation is, for example, 1 to 5, 1 to 3, 1 to 2, or 1. For example, the polynucleotides consisting of the complementary nucleotide sequences include polynucleotides that are hybridized with the polynucleotide consisting of the nucleotide sequence of the miRNA under stringent conditions, and has the function of the miRNA in the present invention. Examples of the stringent conditions include, but not particularly limited to, the conditions described in [0028] of Japanese Patent Application Laid-Open No. 2017-184642, and the contents of this publication are incorporated herein by reference.

In the present invention, it is preferable that the microparticles contain miR-155. It is preferable that the microparticles contain hsa-miR-155-5p among types of miR-155. Furthermore, it is preferable that the microparticles contain hsa-miR-155-5p at higher concentration than culture supernatant of dental pulp-derived stem cells or culture supernatant of adipose-derived stem cells. Hereinafter, preferable embodiments of the microRNA contained in the microparticles will be described.

According to Blood (2012) 119 (15): 3478-3485, miR-155 is induced synergistically upon stimulation of primary human NK cells with IL-12 and IL-18, or IL-12 and CD16 clustering. The overexpression of miR-155 in NK cells increases the induction of IFN-y by the stimulation with IL-12 and IL-18 or CD16. The knockdown of miR-155 or the destruction thereof suppresses the induction of IFN-y by stimulation with monokines or CD16.

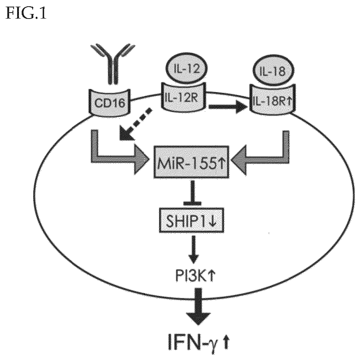

FIG.1 is a schematic diagram showing modeled function of NK cells in the induction of miR-155 and the regulation of production of IFN-y after the activation of the NK cells. Static NK cells are activated with IL-18 alone, CD16 activation alone, or IL-12 alone, followed by the induction of the expression of miR-155. The combination of IL-12 and IL-18 or CD16 activation however induces miR-155 synergistically. This synergistic effect depends on the induction of IL-18R by IL-12. Meanwhile, CD16 and IL-12 synergistically induce miR-155 due to an increase in intercellular signals of downstream of CD16 (broken line arrow). miR-155 targets SHIP1 to reduce the expression of SHIP1, resulting in promoting long-term activation of the PI3K pathway and the subsequent increase in production of IFN-y.

According to Blood (2013) 121 (16): 3126-3134, it is observed that miR-155 transgene (tg) mice increase in the number of NK cells, and improve in NK cell viability. Excess immature CD11blowCD27high NK cells and the activated phenotype thereof are observed. miR-155 tg NK cells are expanded *in vivo* to promote production of interferon g, activation of AKT and ERK, and killing of lymphomas.

Although the overexpression of miR-155 in NK cells is an essential block of terminal differentiation, the overexpression positively controls the expansion and the effector function of NK cells.

If direct administration of the miR-155 oligonucleotide selectively increases miR-155 in NK cells, the administration improves activation of NK cells for treating of intractable cancer or infectious diseases, and may enable avoiding toxicity to which patients are exposed due to systemic administration of cytokine such as IL-2 or IL-12.

SHIP1 is also referred to as 5'-inositol phosphatase or SRC homology 2 domain-containing inositol-5-phosphatase 1.

miRNAs (for example, hsa-miR-155 and hsa-miR-155-5p are known) that targets a gene concerning expression of SHIP1 are effective as agents for activating NK cell.

In the present invention, it is preferable that the microparticles contain miRNA that targets the gene concerning expression of SHIP1, and it is more preferable that the microparticles contain hsa-miR-155-5p as the miRNA that targets the gene concerning expression of SHIP1.

If, in the microparticles of the present invention, the microparticles contain miRNA that targets the gene concerning the expression of SHIP1, the microparticles of the present invention preferably function as an agent for controlling the expression of the gene concerning the expression of SHIP1. That is, it is preferable that the microparticles of the present invention be an agent for controlling the expression of SHIP1.

It is preferable that the microparticles contain at least one of the miRNAs that targets the gene concerning the expression of SHIP1 so that the log2 ratio of the read count obtained by analysis using IMOTA is 8.0 or more; it is more preferable that the microparticles contain at least one of the miRNAs that targets the gene concerning the expression of SHIP1 so that the log2 ratio of the read count is 10.0 or more; and it is particularly preferable that the microparticles contain at least one of the miRNAs that targets the gene concerning the expression of SHIP1 so that the log2 ratio of the read count is 12.0 or more. It is preferable that the microparticles contain hsa-miR-155-5p so that the log2 ratio of the read count obtained by analysis using IMOTA is 8.0 or more; it is more preferable that the microparticles contain hsa-miR-155-5p so that log2 ratio of the read count is 10.0 or more; and it is particularly preferable that the microparticles contain hsa-miR-155-5p so that log2 ratio of the read count is 12.0 or more.

It is preferable that the expression level of hsa-miR-155-5p in the microparticles of the present invention be 1.1 times or more the expression level of hsa-miR-155-5p in exosomes obtained from culture supernatant of umbilical cord-derived stem cells. It is more preferable that the expression level of hsa-miR-155-5p in the microparticles of the present invention be 1.5 times or more the expression level of hsa-miR-155-5p in exosomes obtained from culture supernatant of umbilical cord-derived stem cells. It is particularly preferable that the expression level of hsa-miR-155-5p in the microparticles of the present invention be 2 times or more the expression level of hsa-miR-155-5p in exosomes obtained from culture supernatant of umbilical cord-derived stem cells.

If the microparticles of the present invention is an agent for suppressing the expression of PTX3, it is preferable that the microparticles of the present invention enable reducing the expression of the gene of PTX3 in any cell to 0.8 times or less the normal expression (in untreated cells); it is more preferable that the microparticles of the present invention enable reducing the expression of the gene of PTX3 in any cell to 0.6 times or less the normal expression; and it is particularly preferable that the microparticles of the present invention enable reducing the expression of the gene of PTX3 in any cell to 0.4 times or less the normal expression.

### (Type of miRNAs)

Around 2600 small RNAs are contained in the microparticles derived from culture supernatant of dental pulp-derived stem cells or other cells.

For example, around 1800 of these are miRNAs in dental pulp-derived stem cells. Among these, 180 to 200 miRNAs are contained at high contents. miRNAs contained in the microparticles in the dental pulp-derived stem cells at high contents are characterized by including many miRNAs related to the treatment of cranial nerve disease or others in addition to the activation of NK cells. This was unknown in the past, and is knowledge that the present inventors have newly found. The miRNAs are very different in this feature from miRNAs contained in microparticles in other mesenchymal stem cells at high contents. For example, few microRNAs related to the treatment of cranial nerve disease are included in miRNAs contained in microparticles of bone marrow-derived stem cells at high contents, miRNAs contained in microparticles of adipose-derived stem cells at high contents, or miRNAs contained in microparticles of umbilical cord-derived stem cells at high contents.

### (Type of microparticles)

It is preferable that the microparticles be one type selected from the group consisting of exosomes, fine vesicles, membrane particles, ectosomes and exovesicles, and microvesicles. It is more preferable that the microparticles be exosomes.

It is preferable that the size of the microparticles be 10 to 1000 nm. It is more preferable that the size of the microparticles be 30 to 500 nm. It is particularly preferable that the size of the microparticles be 50 to 150 nm.

It is desirable that molecules such as CD9, CD63, and CD81 referred to as tetraspanins be present on the surfaces of the microparticles. The molecules may be CD9 alone, CD63 alone, CD81 alone, or any combination of two or three thereof.

Although preferable aspects using exosomes as the microparticles may be described hereinafter, the microparticles used for the present invention are not limited to exosomes.

It is preferable that the exosomes be extracellular vesicles released from cells upon the fusion of multivesicular bodies with cytoplasmic membranes.

It is preferable that the surfaces of the exosomes contain lipid and protein derived from cell membranes of dental pulp-derived stem cells.

It is preferable that the exosomes contain intracellular substances such as nucleic acids (for example, microRNAs, messenger RNAs, and DNAs) and proteins in dental pulp-derived stem cells.

It is known that exosomes are used for communication between cells by transport genetic information from the one cell to the other cell. Exosomes can be easily tracked and targeted in a specific region.

### (Content of microparticles)

The content of the microparticles in the microparticle composition is not particularly limited. It is preferable that the microparticle composition contain 0.5 × 10⁸ microparticles or more. It is more preferable that the microparticle composition contain 1.0 × 10⁸ microparticles or more. It is particularly preferable that the microparticle composition contain 2.0 × 10⁸ microparticles or more. It is more particularly preferable that the microparticle composition contain 2.5 × 10⁸ microparticles or more. It is still more preferable that the microparticle composition contain 1.0 × 10⁹ microparticles or more.

The concentration of the microparticles contained in the microparticle composition is not particularly limited. It is preferable that the microparticle composition contain the microparticles at 1.0 × 10⁸ microparticles/mL or more. It is more preferable that the microparticle composition contain the microparticles at 2.0 × 10⁸ microparticles/mL or more. It is particularly preferable that the microparticle composition contain the microparticles at 4.0 × 10⁸ microparticles/mL or more. It is more particularly preferable that the microparticle composition contain the microparticles at 5.0 × 10⁸ microparticles /mL or more. It is still more preferable that the microparticle composition contain the microparticles at 2.0 × 10⁹ microparticles/mL or more.

Since the preferable aspects of the present invention thus contain the microparticles in large amounts or at high concentrations, the amount of the miRNA used for the activation of NK cells can be maintained highly.

### <Other components>

As long as the effect of the present invention is not deteriorated, the microparticle composition may contain other components besides the microparticles depending on the kind of an animal to which the microparticle composition is administered and the purpose. Examples of the other components include nutritional components, antibiotics, cytokines, protectants, carriers, vehicles, disintegrators, buffers, emulsifiers, suspending agents, smoothing agents, stabilizers, preservatives, and antiseptics.

Examples of the nutritional components include fatty acids and vitamins.

Examples of the antibiotics include penicillin, streptomycin, and gentamycin.

Examples of the carriers include ingredients known as pharmaceutically acceptable carriers.

The microparticle composition may be the culture supernatant itself or the microparticles themselves of dental pulp-derived stem cells or other cells, or may be a pharmaceutical composition further containing a pharmaceutically acceptable carrier or a pharmaceutically acceptable vehicle. The pharmaceutical composition is for use in promoting the administration of the microparticles to an administration subject.

It is preferable that the pharmaceutically acceptable carrier (containing a diluent) do not markedly stimulate an administration subject or deter the biological activity or the characteristics of the compound to be administered. Examples of the carrier include propylene glycol; (physiological) saline solution; emulsions; buffer solutions; medium such as DMEM or RPMI; and cryopreservation medium containing a component for removing free radicals.

The microparticle composition may contain a conventionally known active ingredient of a composition containing activated NK cells. Those skilled in the art can appropriately change the active ingredient depending on the purpose or an administration subject.

Meanwhile, it is preferable that the microparticle composition do not contain predetermined substances.

For example, it is preferable that the microparticle composition do not contain dental pulp-derived stem cells or other cells.

It is preferable that the microparticle composition do not contain MCP-1. The microparticle composition may however contain cytokines other than MCP-1. Examples of the other cytokines include cytokines described in [0014] to [0020] of Japanese Patent Application Laid-Open No. 2018-023343.

It is preferable that the microparticle composition do not contain Siglec-9. The microparticle composition may however contain other sialic acid binding immunoglobulin-like lectins than Siglec-9.

It is preferable that the microparticle composition do not substantially contain serum (for example, fetal bovine serum, human serum, or sheep serum). It is preferable that the microparticle composition do not substantially contain conventional serum replacement such as KnockOut serum replacement (KSR).

It is preferable that all the contents (solid content) of the above-mentioned other components in the microparticles be 1% by mass or less. It is more preferable that all the contents be 0.1% by mass or less. It is particularly preferable that all the contents be 0.01% by mass or less.

### <Method for producing microparticles>

The method for producing microparticles is not particularly limited.

Culture supernatant of dental pulp-derived stem cells or other cells may be prepared, followed by purifying microparticles from the culture supernatant to prepare the microparticles of the present invention. Alternatively, culture supernatant of dental pulp-derived stem cells or other cells procured by commercial purchase may be purified to prepare the microparticles of the present invention. Furthermore, a composition that has been discarded and contains culture supernatant of dental pulp-derived stem cells or other cells may be obtained by transfer (and optionally purified), followed by purifying microparticles therefrom to prepare the microparticles of the present invention.

### (Method for preparing culture supernatant of dental pulp-derived stem cells or other cells)

The culture supernatant of the dental pulp-derived stem cells or other cells is not particularly limited.

It is preferable that the culture supernatant of the dental pulp-derived stem cells or other cells do not substantially serum. For example, it is preferable that the content of serum in the culture supernatant of the dental pulp-derived stem cells or other cells be 1% by mass or less. It is more preferable that the content be 0.1% by mass or less. It is particularly preferable that the content be 0.01% by mass or less.

The dental pulp-derived stem cells or other cells may be derived from a human or a non-human animal. Examples of the non-human animal include the same animals (biological species) as the animals to which the microparticles of the present invention are administered. Mammals are preferable.

The dental pulp-derived stem cells used for the culture supernatant are not particularly limited. Stem cells from exfoliated deciduous teeth, deciduous tooth pulp stem cells obtained by other ways, or dental pulp stem cells (DPSCs) are usable. Dental pulp-derived stem cells derived from non-human animals and including pig stem cells from deciduous teeth can be used besides human stem cells from deciduous teeth and human dental pulp stem cells.

Dental pulp-derived stem cells can produce vascular endothelial growth factor (VEGF), hepatocyte growth factor (HGF), insulin-like growth factor (IGF), platelet-derived growth factor (PDGF), transforming growth factorbeta (TGF-β)-1 and -3, TGF-α, KGF, HBEGF, SPARC, other growth factors, cytokines such as chemokines besides exosomes. Dental pulp-derived stem cells can also produce many other bioactive substances.

It is particularly preferable that, in the present invention, the dental pulp-derived stem cells used for the culture supernatant of the dental pulp-derived stem cells contain a large amount of protein. It is preferable that stem cells from exfoliated deciduous teeth be used. That is, it is preferable that, in the present invention, culture supernatant of stem cells from exfoliated deciduous teeth be used.

The adipose-derived stem cells used for the present invention are not particularly limited. Somatic stem cells contained in any adipose tissue can be used as the adipose-derived stem cells. Adipose-derived stem cells such as pig adipose stem cells derived from non-human animals can be used besides human adipose-derived stem cells. Examples of usable adipose-derived stem cells include adipose-derived stem cells prepared by the method described in [0023] to [0041] of International Publication No. WO 2018/038180. The contents of this publication are incorporated herein by reference. Examples of usable adipose-derived stem cells also include adipose-derived stem cells prepared by the method described in [0022] and [0172] to [0187] of Japanese Patent Application Laid-Open No. 2018-531979. The contents of this publication are incorporated herein by reference.

As long as the objective treatment can be achieved, the dental pulp-derived stem cells or other cells used for the present invention may be natural or genetically modified.

Especially in the present invention, immortalized stem cells of the dental pulp-derived stem cells or other cells are usable. The use of immortalized stem cells, which can be substantially proliferated infinitely, enables stabilize the amounts and the composition of biological factors contained in the culture supernatant of the stem cells for a long period of time. The immortalized stem cells of the dental pulp-derived stem cells or other cells are not particularly limited. It is preferable that the immortalized stem cells be not cancerous. To the dental pulp-derived stem cells or other cells can be added the following lowmolecular weight compounds (inhibitors) alone or in combination, followed by culturing the cells to prepare the immortalized stem cells of the dental pulp-derived stem cells or other cells.

As long as a TGF-β receptor inhibitor can inhibit the function of transforming growth factor (TGF)-β receptors, the TGF-β receptor inhibitor is not particularly limited. Examples include 2-(5-benzo[1,3]dioxol-4-yl-2-tert-butyl-1H-imidazol-4-yl)-6-methylpyridine, 3-(6-methylpyridin-2-yl)-4-(4-quinolyl)-1-phenylthiocarbamoyl-1H-pyrazole (A-83-01), 2-[(5-chloro-2-fluorophenyl)pteridin-4-yl]pyridin-4-ylamine (SD-208), 3-[(pyridin-2-yl)-4-(4-quinonyl)]-1H-pyrazole, and 2-(3-(6-methylpyridin-2-yl)-1H-pyrazol-4-yl)-1,5-naphthyridine (the above are available from Merck KGaA); and SB431542 (Merck KGaA). A-83-01 is preferably exemplified.

As long as a ROCK inhibitor has the action of inhibiting the function of Rho-binding kinase, the ROCK inhibitor is not particularly limited. Examples of the ROCK inhibitor include GSK269962A (Axon Medchem), Fasudil hydrochloride (Tocris Bioscience), and Y-27632 and H-1152 (the above are available from FUJIFILM Wako Pure Chemical Corporation). Y-27632 is preferably exemplified.

As long as a GSK-3 inhibitor inhibits GSK-3 (glycogen synthase kinase-3), the GSK-3 inhibitor is not particularly limited. Examples include A 1070722, BIO, and BIO-acetoxime (the above is available from Tocris Bioscience).

As long as a MEK inhibitor has the action of inhibiting the function of MEK (MAP kinase-ERK kinase), the MEK inhibitor is not particularly limited. Examples include AZD6244, CI-1040 (PD184352), PD0325901, RDEA119 (BAY86-9766), SL327, and U0126-EtOH (the above are available from Selleck Biotechnology); and PD98059, U0124, and U0125 (the above are available from Cosmo Bio Co., Ltd.).

If the microparticles of the present invention are used for regenerative medicine, an aspect in which the culture supernatant of the dental pulp-derived stem cells or other cells or immortalized stem cells thereof, or the microparticle composition containing the microparticles derived therefrom does not contain other somatic stem cells except the dental pulp-derived stem cells or other cells is adopted due to request from the Act on Securing Safety of Regenerative Medicine. Although the microparticle composition may contain mesenchymal stem cells or other somatic stem cells except the dental pulp-derived stem cells or other cells, it is preferable that the microparticle composition do not contain these.

Examples of the other somatic stem cells except mesenchymal stem cells include, but not limited to, stem cells derived from the dermal system, the digestive system, the bone marrow system, and the nervous system. Examples of somatic stem cells in the dermal system include epithelial stem cells and follicular stem cells. Examples of somatic stem cells in the digestive system include stem cells of (all types of cells of) the pancreas and hepatic stem cells. Examples of somatic stem cells in the bone marrow system (except mesenchymal stem cells) include hematopoietic stem cells. Examples of somatic stem cells in the nervous system include neural stem cells and retinal stem cells.

Although the microparticle composition may contain stem cells other than somatic stem cells, it is preferable that the microparticle composition do not contain the stem cells other than somatic stem cells. Examples of the stem cells other than somatic stem cells include embryonic stem cells (ES cells), induced pluripotent stem cells (iPS cells), and embryonal carcinoma cells (EC cells).

The method for preparing the culture supernatant of the dental pulp-derived stem cells or other cells or the immortalized stem cells thereof is not particularly limited, and any conventional method is available.

The culture supernatant of the dental pulp-derived stem cells or other cells is culture solution obtained by culturing the dental pulp-derived stem cells or other cells. For example, the dental pulp-derived stem cells can be cultured, followed by removing cell components to obtain the culture supernatant available for the present invention. The culture supernatant may be subjected to various treatments (such as centrifugation, concentration, solvent replacement, dialysis, freezing, drying, lyophilization, dilution, desalting, and preservation) for use.

The dental pulp-derived stem cells for obtaining the culture supernatant of the dental pulp-derived stem cells can be sorted by an ordinary method. The dental pulp-derived stem cells can be sorted as adherent cells based on the sizes or the shapes of the cells. The dental pulp-derived stem cells can be sorted as adherent cells or subcultured cells thereof from dental pulp cells collected from exfoliated deciduous teeth or permanent teeth. The sorted stem cells are cultured to obtain culture supernatant, which is available for the culture supernatant of the dental pulp-derived stem cells.

The adipose-derived stem cells can be sorted as adherent cells or subcultured cells thereof from adipocytes collected from adipose tissue.

It is preferable that the "culture supernatant of the dental pulp-derived stem cells or other cells" do not contain cultured dental pulp-derived stem cells themselves. It is preferable that, in an aspect, the culture supernatant of the dental pulp-derived stem cells or other cells do not contain cells (any types of cells) as a whole. The composition of the aspect is clearly distinguished by this feature from various compositions containing dental pulp-derived stem cells or other cells, let alone from dental pulp-derived stem cells or other cells themselves. A typical example of this aspect is a composition not containing the dental pulp-derived stem cells or other cells and exclusively consisting of the culture supernatant of the dental pulp-derived stem cells or other cells. The culture supernatant of the dental pulp-derived stem cells for the present invention may contain both culture supernatant of dental pulp-derived stem cells from deciduous tooth and culture supernatant of dental pulp-derived stem cells from adults. It is preferable that the culture supernatant of the dental pulp-derived stem cells for the present invention contain culture supernatant of dental pulp-derived stem cells from deciduous teeth as the active ingredient. It is more preferable that the culture supernatant of the dental pulp-derived stem cells for the present invention contain culture supernatant of dental pulp-derived stem cells from deciduous teeth at 50% by mass or more. It is preferable that the culture supernatant of the dental pulp-derived stem cells for the present invention contain culture supernatant of dental pulp-derived stem cells from deciduous teeth at 90% by mass or more. It is particularly preferable that the culture supernatant of the dental pulp-derived stem cells for the present invention be a composition exclusively consisting of culture supernatant of dental pulp-derived stem cells from deciduous teeth.

For example, basal medium or basal medium containing serum and others are available as medium for the dental pulp-derived stem cells or other cells for obtaining the culture supernatant. Examples of the available basal medium include Iscove's modified Dulbecco's medium (IMDM) (for example, Thermo Fisher Scientific K.K.), Ham's F12 medium (HamF12) (for example, Merck KGaA or Thermo Fisher Scientific K.K.), and RPMI 1640 medium besides Dulbecco's modified Eagle medium (DMEM). Examples of components that can be added to the medium include sera (for example, fetal bovine serum, human serum, and sheep serum), serum replacements (for example, KnockOut serum replacement (KSR)), bovine serum albumin (BSA), antibiotics, various vitamins, and various minerals.

In order to prepare "the culture supernatant of the dental pulp-derived stem cells or other cells" not containing serum, serum-free medium is however preferably used through the whole process, during the last subculture, or during the last several subcultures. For example, the dental pulp-derived stem cells or other cells can be cultured in medium not containing serum (serum-free medium) to prepare the culture supernatant of the dental pulp-derived stem cells or other cells not containing serum. During one or more subcultures, the last subculture or the last several subcultures using serum-free medium also enable obtaining the culture supernatant of the dental pulp-derived stem cells or other cells not containing serum. Meanwhile, the serum can also be removed from the collected culture supernatant by dialysis or solvent replacement using a column to obtain the culture supernatant of the dental pulp-derived stem cells or other cells not containing serum.

Conditions to be commonly used can be applied to culture of the dental pulp-derived stem cells or other cells for obtaining the culture supernatant as they are. The culture supernatant of the dental pulp-derived stem cells or other cells only have to be prepared in the same way as in the below-described method for culturing cells except the adjustment of the steps of isolating and selecting the stem cells depending on the type of the stem cells. Those skilled in the art can suitably isolate and select the dental pulp-derived stem cells or other cells depending on the type thereof.

Special conditions may be applied to the culture of the dental pulp-derived stem cells or other cells for producing a large amount of the microparticles such as exosomes. Examples of the special conditions include low temperature conditions, low oxygen conditions, microgravity conditions, and conditions for coculture with some stimulant.

Although, in the present invention, the culture supernatant of the dental pulp-derived stem cells or other cells for preparing the microparticles such as exosomes may contain other components besides the culture supernatant of the dental pulp-derived stem cells or other cells, it is preferable that the culture supernatant do not substantially contain the other components.

Various additives for preparing the exosomes may however be added to the culture supernatant of the dental pulp-derived stem cells or other cells for storage.

### (Preparation of microparticles)

The microparticles can be purified and prepared from the culture supernatant of the dental pulp-derived stem cells or other cells.

It is preferable that the preparation of the microparticles be the separation of fractions containing the microparticles from the culture supernatant of the dental pulp-derived stem cells or other cells. It is more preferable that the preparation be the isolation of the microparticles.

The microparticles can be separated from non-associated components based on characteristics of the microparticles, resulting in isolation. For example, the microparticles can be isolated based on the molecular weight, the size, the shape, the composition, or the biological activity.

In the present invention, the culture supernatant of the dental pulp-derived stem cells or other cells can be centrifuged to obtain specific fractions containing the microparticles in a large amount (for example, precipitate), which fractions can be collected to purify the microparticles. Unnecessary components (insoluble components) other than the predetermined fractions may be removed. The solvent and the dispersion medium, and the unnecessary components may not be fully removed from the microparticle composition. If the conditions for centrifugation are exemplified, the conditions are 100 to 20000 g and 1 to 30 minutes.

In the present invention, the culture supernatant of the dental pulp-derived stem cells or other cells or the centrifuged product thereof can be filtered to purify the microparticles. The filtration enables removing the unnecessary components. The use of a membrane filter having a suitable pore size enables removing the unnecessary components and sterilization simultaneously. The material, the pore size and other properties of the membrane filter used for filtration are not particularly limited. The microparticles can be filtered off on a filter membrane of a molecular weight or size cutoff by a conventionally known method. It is preferable from the viewpoint of collecting exosomes easily that the pore size of the filter membrane be 10 to 1000 nm. It is more preferable that the pore size be 30 to 500 nm. It is particularly preferable that the pore size be 50 to 150 nm.

In the present invention, the culture supernatant of the dental pulp-derived stem cells or other cells, the centrifuged product thereof, or the filtered product thereof can be separated with further separation means such as column chromatography. For example, high-performance liquid chromatography (HPLC) using various columns is available. Size-exclusion columns or binding columns are available as the columns.

One or more characteristics or biological activities of the microparticles are usable for tracking the microparticles (or the activity thereof) in fractions in the treatment steps. For example, light scattering, the index of refraction, dynamic light scattering, or an UV-visible light detector are usable for tracking the microparticles. Alternatively, specific enzyme activity is usable for tracking the activity in the fractions.

The method described in [0034] to [0064] of Japanese Translation of PCT International Application Publication No. 2019-524824 may be used as the method for purifying the microparticles. The contents of this publication are incorporated herein by reference.

The final form of the microparticles is not particularly limited. Examples of the form include a form in which a container is filled with the microparticles together with a solvent or a dispersion medium; a form in which a container is filled with gel prepared from the microparticles and gel; and a form of a preparation made from the microparticles solidified by freezing and/or drying; or a form in which a container is filled with the solidified microparticles. Examples of the container include tubes, centrifuge tubes, and bags suitable for cryopreservation. The freezing temperature can be, for example, -20°C to -196°C.

The microparticles of the present invention are advantageous in that the microparticles of the present invention are easily mass-produced as compared with conventional compositions usable as agents for activating NK cells, culture supernatant of stem cells, which culture supernatant was discarded as industrial waste in the past, is utilizable, and cost for discarding culture supernatant of stem cells can be reduced. Especially if the culture supernatant of the dental pulp-derived stem cells or other cells is derived from a human, the microparticles of the present invention are also advantageous in that, upon the application of the microparticles of the present invention to a human, the microparticles of the present invention are highly safe from immunological viewpoint, and have few ethical problems. If the culture supernatant of the dental pulp-derived stem cells or other cells is collected from a healthy person or a patient who desires the activation of NK cells, this will enhance the safety of the microparticles of the present invention and reduce ethical problems of the microparticles of the present invention upon the application of the microparticles of the present invention to the patient.

If the microparticles of the present invention are derived from the culture supernatant of the dental pulp-derived stem cells or other cells, the microparticles of the present invention are also used for restorative medicine. Especially the composition containing the microparticles of the present invention derived from the culture supernatant of the dental pulp-derived stem cells or other cells are preferably used for restorative medicine. It is known that, in regenerative medicine, premised on stem cell implantation, the stem cells do not play the leading role in regeneration, and humoral components produced in the stem cells restore organs together with the subject's own stem cells. Difficult problems are solved including canceration, standardization, the administration method, the preservation, and the culture method accompanying conventional stem cell plantation, so that restorative medicine can be performed with the culture supernatant of the dental pulp-derived stem cells or other cells or the composition containing the microparticles derived therefrom. Since the use of the microparticles of the present invention does not involve cell plantation, neoplastic transformation is less likely to be caused by the use thereof than by stem cell plantation, so that the use thereof is safer than stem cell plantation. The microparticles of the present invention are advantageous in that the microparticles of the present invention can be used having quality standardized uniformly. Since the mass production or effective administration method thereof can be selected, the microparticles of the present invention is available at low cost.

### [Agent for activating NK cells]

An agent for activating NK cells of the present invention contains the microparticles of the present invention.

The agent for activating NK cells of the present invention may exclusively consist of the microparticles of the present invention or contain components other than the microparticles of the present invention.

One of the preferable aspects of the agent for activating NK cells of the present invention is an agent for activating NK cells exclusively consisting of culture supernatant of dental pulp-derived stem cells or culture supernatant of adipose-derived stem cells.

The agent for activating NK cells is however allowed to contain inevitable components in a minute amount (at preferably 1% by mass or less, more preferably 0.1% by mass or less, particularly preferably 0.01% by mass or less).

### [Method for culturing NK cells]

In a method for culturing NK cells of the present invention, NK cells are cultured in medium for activating NK cells containing an effective amount of the microparticles of the present invention or an effective amount of the agent for activating NK cells of the present invention.

The medium used for medium for activating NK cells is not particularly limited, and any known medium and commercially available product thereof may be used. Examples of the usable medium include the serum-free lymphocyte medium X-VIVO 15 and the serum-free lymphocyte medium X-VIVO 20 (all are available from Lonza K.K., and X-VIVO is a registered trademark.)

The NK cells are not particularly limited, and any known NK cells and commercially available product thereof may be used. For example, NK cells (available from Lonza K.K.) isolated from human peripheral blood or mononuclear cell fractions is usable. NK cells acquired, for example, from peripheral blood of an animal such as a human may be used.

The condition for culturing NK cells is not particularly limited, and any known condition may be used. For example, the culture temperature can be 35 to 40°C. It is preferable that the culture temperature be 36 to 38°C. The culture time can be 1 hour to 30 days. It is preferable that the culture time be 12 hours to 20 days. It is more preferable that the culture time be 1 to 10 days.

The effective amount of the microparticles of the present invention or the agent for activating NK cells of the present invention for the culture of NK cells is not particularly limited, and the effective amount can be suitably adjusted depending on the origin of the microparticles. In the present invention, it is preferable that the number of the microparticles per one NK cell be 5 or more. It is more preferable that the number be 50 or more. It is particularly preferable that the number be 100 or more. It is more particularly preferable that the number be 500 or more. Especially if exosomes derived from adipose-derived stem cells are used, it is particularly preferable that the number of the microparticles per one NK cells be 500 or more. It is more preferable that the number be 800 or more. It is particularly preferable that the number be 1000 or more.

### [Method for producing activated NK cells]

A method for producing activated NK cells of the present invention includes administering an effective amount of the microparticles of the present invention or an effective amount of the agent for activating NK cells of the present invention to a composition containing NK cells.

Preferable embodiments of the method for producing activated NK cells of the present invention are similar to the preferable embodiments of the NK cells of the present invention.

The purpose for which the produced activated NK cells are used is not particularly limited. For example, the produced activated NK cells may be used for testing or investigating activated NK cells or for (high level) activated NK cell therapy.

### [Method for activating NK cells]

A method for activating NK cells of the present invention includes administering an effective amount of the microparticles of the present invention or an effective amount of the agent for activating NK cells of the present invention to a subject.

The step of administering the microparticles or the agent for activating NK cells of the present invention to a subject is not particularly limited.

Examples of the administration method include the nebulization or the inhalation into the oral cavity, the nasal cavity, or the respiratory tract; an intravenous drip; local administration; and nose drops. It is preferable that the administration method be less invasive. Injection is preferable as the local administration. Electroporation is also preferable for the following reason: Electroporation enables applying a voltage (electrical pulse) to the surface of the skin to bore fine pores in cell membranes to make active ingredients permeate into the dermal layer, which is unapproachable by ordinary care. Examples of the local administration include intravenous administration, intraarterial administration, portal venous administration, intradermal administration, subcutaneous administration, intramuscular injection, and intraperitoneal administration. It is preferable that the local administration be intraarterial administration, intravenous administration, subcutaneous administration, or intraperitoneal administration. The *in vivo* distribution of the microparticles or the agent for activating NK cells can be changed by various formulation techniques. Many methods for changing the *in vivo* distribution are known to those skilled in the art. Examples of such methods include the protection of exosomes in vesicles formed of a substance such as protein, lipid (liposomes), carbohydrate, or a synthesized polymer.

The microparticles or the agent for activating NK cells of the present invention administered to a subject may be circulated in the body to reach predetermined tissue. Since it is known that many NK cells are present in the intestine, it is preferable that the microparticles or the agent for activating NK cells reaches the intestine.

The number of times of administration and administration interval are not particularly limited. The number of times of administration can be one or more times per week. It is preferable that the number be 5 or more. It is more preferable that the number be 6 or more. It is particularly preferable that the number be 7 or more. It is preferable that the administration interval be 1 hour to 1 week. It is more preferable that the administration interval be half a day to 1 week. It is particularly preferable that the administration interval be 1 day (once daily). The administration interval can however be suitably adjusted depending on the biological species of the administration subject or the symptom(s) of the administration subject.

It is preferable that the microparticles or the agent for activating NK cells of the present invention is for use in the administration thereof one or more times per week during the therapeutic effective period. If the administration subject is a human, it is preferable that the number of times of administration per week be more. It is preferable that the number of times of administration be 5 times or more per week during the therapeutic effective period. Daily administration is preferable.

If the culture supernatant of the dental pulp-derived stem cells or other cells are used having a concentration of 2.0 × 10⁹ to 2.0 × 10⁹ microparticles/ml, with respect to a mouse model, it is preferable that the volume of the culture supernatant be 0.1 to 5 ml per mouse (around 25 g), it is more preferable that the volume be 0.3 to 3 ml, and it is particularly preferable that the volume be 0.5 to 1 ml.

If the microparticles are used having a concentration of 0.1 × 10⁸ microparticles/µg, with respect to a mouse model, it is preferable that the weight of the microparticles be 1 to 50 µg per mouse (around 25 g), it is more preferable that the weight be 3 to 30 µg, and it is more particularly preferable that the weight be 5 to 25 µg.

A preferable range of the dose to another animal per the body weight of the other animal can be calculated in proportion to the dose to the model mouse per the body weight of a model mouse (around 25 g). The dose can however be suitably adjusted depending on the symptom(s) of an administration subject.

In the present invention, the number of the microparticles per one NK cell of the subject may be calculated from the concentration of the NK cells of the subject to determine the dose of the microparticles or the agent for activating NK cells. Preferable aspects of the number of the microparticles per one NK cell in the method for activating NK cells are the same as preferable aspects of the number of the microparticles per one NK cell in the method for culturing NK cells. In the present invention, it is preferable that, for example, the number of the microparticles per one NK cell be 50 or more.

The subject animal (biological species) to which the microparticles or the agent for activating NK cells of the present invention are administered is not particularly limited. It is preferable that the microparticles of the present invention be administered to a mammal, a bird (for example, a chicken, a quail, or a duck), a fish (for example, a salmon, a trout, a tuna, or a bonito) as a subject animal. The mammal may be a human or a non-human mammal. It is particularly preferable that the mammal be a human. It is more preferable that the non-human mammal be a bovine, a pig, a horse, a goat, a sheep, a simian, a dog, a cat, a mouse, a rat, a guinea pig, or a hamster.

In one of the preferable aspects, the microparticles or the agent for activating NK cells of the present invention is administered to a non-human animal as a subject animal.

The microparticles or the agent for activating NK cells of the present invention may be used in combination with a conventionally known agent for activating NK cells. The microparticles or the agent for activating NK cells of the present invention may be specifically used, for example, in combination with a conventionally known immunosuppressant or biopharmaceutical (IFN-y enhancer), IL-2, IL-12, IL-18, or CD16 clustering.

### <Use of microparticles>

The present invention may be the use of the microparticles of the present invention or the agent for activating NK cells of the present invention in an effective amount for activating NK cells in the living body of a subject. That is, the present invention may be the use of the microparticles of the present invention for activating NK cells in the living body of a subject, the microparticles containing miRNA with respect to the activation of NK cells and being derived from the culture supernatant of the dental pulp-derived stem cells or the culture supernatant of the adipose-derived stem cells.

The present invention may be the use of the microparticles of the present invention for promoting granzyme B in NK cells in the living body of a subject.

The present invention may be the use of the microparticles of the present invention for controlling the expression of SHIP1 in NK cells in the living body of a subject.

### Examples

Hereinafter, features of the present invention will be described further specifically by giving Examples and Comparative Examples or Reference Examples. For example, materials, the used amounts, the ratios, the contents of treatments, and the procedures of the treatments shown in the following Examples can be optionally modified as long as these do not depart from the gist of the present invention. Accordingly, the scope of the present invention should not be interpreted to be limited by specific examples shown hereinafter.

### [Example 1]

### <Preparation of culture supernatant of dental pulp-derived stem cells>

Culture supernatant of human stem cells from deciduous teeth was prepared and collected according to the method described in Example 6 of Japanese Patent No. 6296622 except that DMEM medium was substituted for DMEM/HamF12 medium. Fetal bovine serum (FBS) was added to the cells, which were primarily cultured. The primary culture solution was cultured to obtain subculture solution. The supernatant thereof was collected so that FBS was not contained. The culture supernatant of the human stem cells from the deciduous teeth was prepared. Note that the DMEM is Dulbecco's modified Eagle medium, and the F12 is Ham's F12 medium.

### <Preparation of exosomes>

Exosomes of the dental pulp-derived stem cells were purified from the obtained culture supernatant of the dental pulp-derived stem cells by the following method.

The culture supernatant of the human stem cells from the deciduous teeth (100 mL) was filtered through a filter having a pore size of 0.22 micrometers, and the solution was then centrifuged at 100000 g and 4°C for 60 minutes. The supernatant was decanted. The exosome-concentrated pellet was resuspended in phosphate-buffered saline (PBS). The resuspended sample was centrifuged at 100000 g for 60 minutes. The pellet (around 100 µl) was collected from the bottom of the centrifuge tube as a concentrated sample again. The protein concentration was determined with a Micro BSA Protein Assay Kit (Thermo Fisher Scientific K.K., Rockford, IL). The composition (concentrated solution) containing the exosomes was stored at -80°C.

The composition containing the exosomes purified from the culture supernatant of the dental pulp-derived stem cells was used as the microparticle composition sample of Example 1.

Evaluated were the average particle size and the concentration of the microparticles contained in the microparticle composition sample of Example 1.

The microparticles contained in the microparticle composition sample of Example 1 had an average particle size of 50 to 150 nm.

The microparticle composition of Example 1 was an exosome solution at a high concentration of 1.0 × 10⁹ exosomes/ml or more, specifically 2.3 × 10⁹ exosomes/ml.

The components of the obtained microparticle composition of Example 1 were analyzed by a known method, which consequently has shown that microparticle composition of Example 1 does not contain the dental pulp-derived stem cells, MCP-1, or Siglec-9. This therefore has shown that the active ingredients of the microparticle composition of Example 1 are different from MCP-1 and Siglec-9, which were active ingredients of culture supernatant of mesenchymal stem cells, and analogs thereof.

### <Test Example 1> microRNAs expressed in exosomes

small RNAs contained in the microparticle composition of Example 1 were analyzed by next generation sequencing (NGS). The NGS analysis has identified 1787 microRNAs contained in the microparticle composition (exosomes of the dental pulp-derived stem cells) of Example 1. The obtained results are shown in the following Table 1.

**[Table 1]**

| name | The number of RNAs detected | % |
|---|---|---|
| miRNA | 1787 | 72.35 |
| piRNA | 123 | 4.98 |
| tRNA | 411 | 16.64 |
| other RNA | 149 | 6.03 |
| Total | 2470 | 100 |

### <Test Example 2> Exploration of microRNAs related to the activation of NK cells

microRNAs concerning diseases were explored. The microRNAs related to diseases were sampled by IMOTA (interactive multi-omics-tissue atlas). IMOTA is an interactive multi-omics atlas that enables investigating the interactions between miRNAs or mRNAs and proteins and the expression levels thereof in tissues or cells (Nucleic Acids Research, Volume 46, Issue D1, 4 January 2018, Pages D770-D775, "IMOTA: an interactive multi-omics tissue atlas for the analysis of human miRNA-target interactions"). It was explored whether the microparticles of the present invention contained microRNAs that control proteins and/or genes related to the activation of NK cells. This Test Example 2 explored miRNAs that target the gene concerning the expression of SHIP1 as a protein involved in the activation of NK cells.

Consequently, it was found that the microparticles of the present invention contained hsa-miR-155-5p as the miRNA that targets the gene concerning the expression of SHIP1. The expression level of hsa-miR-155-5p was 12.17 as the log2 ratio of the read count obtained by analysis using IMOTA. This was the 44th out of the expression levels of the 1787 microRNAs, which were all of the miRNAs contained in the microparticle composition (exosomes of the dental pulp-derived stem cells) of Example 1 shown in Table 1.

This has therefore shown that since the microparticles of the present invention contains hsa-miR-155-5p in a large amount, the microparticles of the present invention are used as an agent for controlling the expression of SHIP1.

The lowest value (limit of detection) of the log2 ratio of the read count was 3.32 among those of the 1787 miRNAs in Table 1. Among the 1787 miRNAs in Table 1, the log2 ratios of the read counts were 4 or more in 1204 miRNAs, the log2 ratios of the read counts were 6 or more in 375 miRNAs, the log2 ratios of the read counts were 8 or more in 178 miRNAs, the log2 ratios of the read counts were 10 or more in 90 miRNAs, the log2 ratios of the read counts were 12 or more in 48 miRNAs, and the log2 ratios of the read counts were 14 or more in 15 miRNAs.

The above has shown that the microparticles of the present invention contain miRNA (hsa-miR-155-5p) related to the activation of NK cells at high concentration. The microparticles of the present invention therefore enable controlling the expression of proteins and/or genes related to the activation of NK cells. Consequently, it has been found that the microparticles of the present invention are useful for the activation of NK cells.

### <Test Example 3> Evaluation of activation of NK cells

The measurement of granzyme B derived from NK cells evaluated the activation of NK cells using the exosomes derived from the mesenchymal stem cells.

NK cells (available from Lonza K.K., the number of the cells: 5 × 10⁶ cells) isolated from mononuclear cell fractions of human peripheral blood were used as the NK cells. X-VIVO 15 serum-free lymphocyte medium (available from Lonza K.K.) was used as the medium.

The NK cells were seeded in 10 ml of the medium in a system for culture in a T75 flask. The microparticle composition of Example 1 was further added thereto so that the number of the exosomes per one NK cell (the number of exosomes for treatment) was 10. The NK cells were cultured. Since the microparticle composition of Example 1 contained 2.3 × 10⁹ exosomes, the percentage of the microparticle composition of Example 1 added to the medium was around 0.5% by volume (around 0.05 ml of the microparticle composition relative to 10 ml of the medium).

With reference to Lonza K.K., the culture conditions for activation were common conditions, specifically 37°C and 5 days (also refer to, for example, [0059] to [0071] of Japanese Patent Application Laid-Open No 2014-030375).

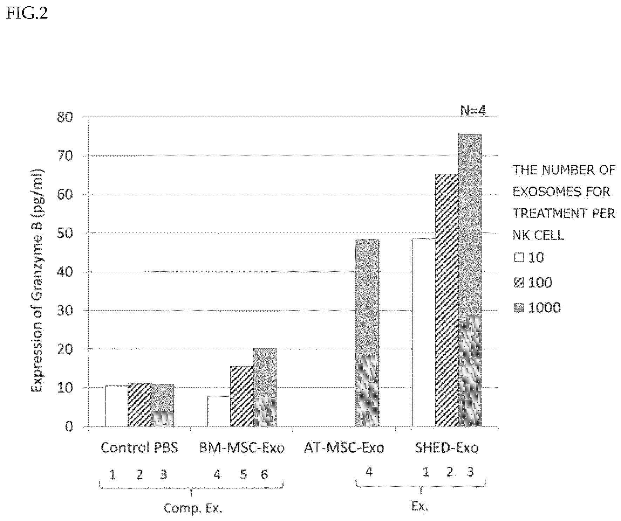

The NK cells cultured separately were measured for the expression level of granzyme B with SensoLyte 520 Granzyme B Activity Assay Kit, Fluorimetric (available from AnaSpec) according to the protocol attached to the kit. The obtained results have been shown in FIG. 2 (the left column of SHED-Exo).

### [Examples 2 and 3]

The expression levels of granzyme B were measured in the same way as in Example 1 except that, in Test Example 3, the microparticle composition of Example 1 was added so that the number of the microparticles per one NK cell (the number of exosomes for treatment) was 100 and 1000 to culture the NK cells of Examples 2 and 3, respectively. The obtained results have been shown in FIG. 2 (the central and right columns of SHED-Exo).

In Example 2, the percentage of the microparticle composition of Example 1 added to the medium was around 5% by volume (around 0.5 ml of the microparticle composition relative to 10 ml of the medium). In Example 3, the percentage of the microparticle composition of Example 1 added to the medium was around 50% by volume (around 5 ml of the microparticle composition relative to 10 ml of the medium).

### [Example 4]

### <Preparation of culture supernatant of adipose-derived stem cells>

Culture supernatant of adipose-derived stem cells was prepared according to Example 1 except that human adipose-derived stem cells were substituted for the human stem cells from the deciduous teeth. The culture supernatant of the adipose-derived stem cells contains exosomes derived from the culture supernatant of the adipose-derived stem cells (AT-MSC-Exo).

### <Evaluation of activation of NK cells>

The expression level of granzyme B was measured in the same way as in Examples 1 to 3, respectively, except that, in Test Example 3, the culture supernatant of the adipose-derived stem cells was added so that the number of the exosomes per one NK cell (the number of exosomes for treatment) was 1000 instead of the microparticle composition of Example 1 to culture the NK cells of Example 4. The obtained results have been shown in FIG. 2 (AT-MSC-Exo).

### [Comparative Examples 1 to 3]

The expression levels of granzyme B were measured in the same way as in Examples 1 to 3, respectively, except that, in Test Example 3, phosphate-buffered saline (PBS (-)) was added in the same added amounts as in Examples 1 to 3, respectively, followed by the culture of NK cells of Comparative Examples 1 to 3 as controls. The obtained results have been shown in FIG. 2 (Control PBS).

### [Comparative Examples 4 to 6]

### <Preparation of culture supernatant of bone marrow-derived stem cells>

Culture supernatant of bone marrow-derived stem cells was prepared according to Example 1 except that human bone marrow-derived stem cells were substituted for stem cells from deciduous teeth. The culture supernatant of the bone marrow-derived stem cells contains exosomes derived from the culture supernatant of the bone marrow-derived stem cells (BM-MSC-Exo).

### <Evaluation of activation of NK cells>

The expression levels of granzyme B were measured in the same way as in Examples 1 to 3 except that, in Test Example 3, the culture supernatant of the bone marrow-derived stem cells was added so that the number of the exosomes per one NK cell (the number of exosomes for treatment) was 10, 100 or 1000 instead of the microparticle composition of Example 1 to culture the NK cells of Comparative Examples 4 to 6, respectively. The obtained results have been shown in FIG. 2 (BM-MSC-EXO).

FIG. 2 has shown that the microparticles derived from the culture supernatant of the dental pulp-derived stem cells of Examples 1 to 3 and the microparticles derived from the culture supernatant of the adipose-derived stem cells of Example 4 enable controlling the expression of the protein and/or the gene related to the activation of NK cells to activate NK cells. It has been shown especially that these microparticles are an agent for promoting the expression of granzyme B.

It has been shown that, especially in Examples 2 and 3, NK cells can be activated by adjusting the number of the microparticles derived from the culture supernatant of the dental pulp-derived stem cells per one NK cell to 100 or more.

Meanwhile, Comparative Examples 1 to 3 have shown that if the amount of PBS added is changed, the expression of granzyme B is not changed, resulting in no change in the activation of NK cells. It has been shown that the microparticles derived from the culture supernatant of the dental pulp-derived stem cells of Comparative Examples 4 to 6 do not enable promoting the expression of granzyme B, namely activating NK cells, remarkably.

## Claims

1. Microparticles, comprising:
miRNA related to activation of NK cells,
wherein the microparticles are derived from culture supernatant of dental pulp-derived stem cells or culture supernatant of adipose-derived stem cells.

2. The microparticles according to claim 1, wherein the microparticles are exosomes.

3. The microparticles according to claim 1, wherein the microparticles are an agent for promoting expression of granzyme B.

4. The microparticles according to claim 1,
wherein the microparticles are an agent for controlling expression of SHIP1,
and comprise hsa-miR-155-5p as miRNA targeting a gene concerning expression of SHIP1.

5. The microparticles according to claim 1, wherein the microparticles are isolated by purification from the culture supernatant of the dental pulp-derived stem cells or the culture supernatant of the adipose-derived stem cells.

6. The microparticles according to claim 5, wherein the microparticles do not comprise components of the culture supernatant of the dental pulp-derived stem cells or the culture supernatant of the adipose-derived stem cells other than the microparticles.

7. The microparticles according to claim 1, comprising:
hsa-miR-155-5p at higher concentration than the culture supernatant of the dental pulp-derived stem cells or the culture supernatant of the adipose-derived stem cells.

8. An agent for activating NK cells, comprising:
the microparticles according to claim 1.

9. A method for culturing NK cells, wherein NK cells are cultured in medium for activating NK cells comprising an effective amount of the microparticles according to claim 1 or an effective amount of the agent for activating NK cells according to claim 8.

10. A method for producing activated NK cells, comprising:
administering an effective amount of the microparticles according to claim 1 or an effective amount of the agent for activating NK cells according to claim 8 to a composition comprising NK cells.

11. NK cells
cultured in medium for activating NK cells comprising an effective amount of the microparticles according to any one of claim 1 or an effective amount of the agent for activating NK cells according to claim 8; or
produced by administering an effective amount of the microparticles according to claim 1 or an effective amount of the agent for activating NK cells according to claim 8 to a composition comprising the NK cells.

12. A method for activating NK cells, comprising:
administering an effective amount of the microparticles according to claim 1 or an effective amount of the agent for activating NK cells according to claim 8 to a subject.

13. The method for activating NK cells according to claim 12, wherein the subject is a non-human animal.

14. The method for activating NK cells according to claim 12, wherein the number of the microparticles per one NK cell is 50 or more.
